# EUROPÄISCHE PATENTSCHRIFT

(11) **EP 2 134 315 B1**
(45) Veröffentlichungstag und Bekanntmachung des Hinweises auf die Patenterteilung: **09.11.2016**
(21) Anmeldenummer: 08748749.2
(22) Anmeldetag: 15.04.2008
(51) Int. Cl.: A61K 8/33, A61K 8/22, A61K 8/365, A61K 8/37, A61Q 19/02, A61K 8/41, A61K 8/49, A61Q 1/14

(54) **ZUSAMMENSETZUNG ZUR ENTFERNUNG EINER TÄTOWIERUNG**
TATTOO-REMOVING COMPOSITION
COMPOSITION POUR ENLEVER UN TATOUAGE

(30) Priorität: 19.04.2007 DE 102007018886; 12.07.2007 DE 102007032844
(43) Veröffentlichungstag der Anmeldung: 23.12.2009
(73) Patentinhaber: SK ageless-concept GmbH, 24536 Neumünster (DE)
(72) Erfinder: SCHIBILLA, Lothar, 24536 Neumünster (DE)
(74) Vertreter: Lau-Loskill, Philipp
(86) Internationale Anmeldenummer: PCT/DE2008/000633
(87) Internationale Veröffentlichungsnummer: WO 2008/128514

(56) Entgegenhaltungen:
- WO-A-89/12440
- DE-A1- 10 056 114
- JP-A- 2003 339 875
- US-A1- 2005 019 355
- US-A1- 2006 052 266

## Beschreibung

Die vorliegende Erfindung betrifft zwei Zusammensetzungen zur Entfernung einer auf einem Hautbereich vorhandenen Tätowierung mit den Merkmalen der Oberbegriffe der Patentansprüche 1 und 9.

Abhängig vom jeweiligen Modetrend oder zur sonstigen Kennzeichnung werden auf dem menschlichen Körper Tätowierungen nach unterschiedlichsten Techniken angebracht, wobei oftmals beim Träger der Wunsch besteht, nach einer gewissen Zeit diese Tätowierungen wieder zu entfernen oder durch andere, neuere Tätowierungen zu ersetzen.

Um diese Tätowierungen zu erzeugen, wird in der Regel die Haut an einem ausgewählten Hautbereich mit einer oder mehreren Nadeln punktiert, um so einen Farbstoff, bei dem es sich in der Regel um ein Farbpigment handelt, in die Haut einzubringen. Während dieses Tätowierungsvorganges ist darauf zu achten, daß zur Erzeugung einer dauerhaften Tätowierung der Farbstoff, bei dem es sich auch um mehrere Farbstoffe handeln kann, so in der Haut deponiert wird, daß dieser Farbstoff weder zu oberflächlich noch zu tief lokalisiert ist. Im ersten Falle würde der so in die Haut eingelagerte Farbstoff infolge des ständigen Erneuerns der oberen Hautschichten auswachsen, so daß nach einer gewissen Zeit die Tätowierung unerwünscht verschwunden wäre, während bei einem zu tiefen Einbringen des Farbstoffes in die Haut Blutungen auftreten können, die zu einem Auswaschen des Farbstoffes führt. Von daher ist es erforderlich, daß für die Herstellung von dauerhaften Tätowierungen die jeweils ausgewählten Farbstoffe bzw. Farbpigmente in eine mittlere Hautschicht positioniert werden.

Ergänzend ist noch darauf hinzuweisen, daß neben den zuvor beschriebenen überwiegend professionell erzeugten Tätowierungen noch Tätowierungen bekannt sind, die mit anderen farbigen Substanzen, so zum Beispiel Asche oder Tinte, erzeugt werden oder die infolge der Verunreinigung einer Wunde zustande kommen, wobei sich bei der letzteren Möglichkeit insbesondere farbige Schmutzpartikel in der Lederhaut ablagern können. Auch derartige Tätowierungen, die laienhaft erzeugt werden oder unerwünscht infolge einer Wundverschmutzung entstehen, sollen im Rahmen der vorliegenden Anmeldung zusammen mit den eingangs beschriebenen professionell erzeugten Tätowierungen kurz als Tätowierung zusammengefaßt werden.

Um derartige Tätowierungen zu entfernen, ist es bekannt, diese Tätowierungen operativ herauszuschneiden oder durch Abschälen der Hautschichten oder mittels einer Laserbehandlung zu beseitigen.

So beschreibt beispielsweise die EP 1 330 199 B eine Vorrichtung zur Entfernung von Tätowierungen, die im wesentlichen darauf abstellt, daß die die Tätowierung ausbildenden Farbstoffpigmente mittels Nadeln, die mit einer konstanten Stichfrequenz vibrieren, mechanisch zerstört werden, so daß dann diese mechanisch zerstörten Farbstoffpigmente zusätzlich noch durch Hautreizstoffe, die auf die Hautoberfläche aufgebracht werden, ausgeschwemmt werden können. Hier nennt die EP 1 330 199 B als Hautreizstoffe wäßrige Lösungen von Milchsäure, Natriumhydracin, Kochsalz, Aminosäuren, wobei diese einzelnen Hautreizstoffe ggf. mit geringen Mengen an Oxidationsmitteln versetzt sein können.

Die zuvor beschriebenen bekannten Verfahren, insbesondere auch die operativen Verfahren oder die Laserbehandlung, haben jedoch den Nachteil, daß häufig in dem tätowierten Hautbereich nach Entfernung der Tätowierung eine mehr oder weniger deutlich sichtbare Narbenbildung auftritt, so daß dieser Hautbereich einerseits ästhetisch störend wirkt und andererseits nicht mehr zur Verfügung steht, um entsprechend neue Tätowierungen anzubringen, was vielfach dem Wunsch der heutigen Zeit entspricht.

Der vorliegenden Erfindung liegt die Aufgabe zugrunde, eine Zusammensetzung zur Verfügung zu stellen, mit der es möglich ist, eine narbenfreie Entfernung einer Tätowierung zur Verfügung zu stellen.

Diese Aufgabe wird erfindungsgemäß durch eine Zusammensetzung mit den kennzeichnenden Merkmalen des Patentanspruchs 1 und/oder mit den kennzeichnenden Merkmalen des Patentanspruchs 9 gelöst.

Die erfindungsgemäße Zusammensetzung zur Entfernung einer auf einem Hautbereich vorhandenen Tätowierung nach vorheriger Enthaarung, Desinfektion und einer Behandlung zum mindestens teilweise Abtragen der Epidermis im Bereich der Tätowierung, insbesondere zum teilweise Abtragen der Hornhaut im Bereich der Tätowierung, sieht vor, daß die erfindungsgemäße Zusammensetzung eine flüssige bis gelartige Konsistenz aufweist und als Wirkstoff eine Mischung aus
- a): Wasser,
- b): Milchsäureethylester,
- c): 4-Hydroxy-2-nonenal (HNE) und/oder d-Erythro-2-aminooctadec-4-en-1,3-diol und/oder Trihydroxypalmitamidohydroxypropyl Myristyl Ester,
- d): 4-Hydroxybutyl-Propionsäure,
- e): 2-Hydroxybutyl-Propionsäure,
- f): 2-Trihydroxypurin sowie
- g): Wasserstoffperoxid
enthält.

Überraschend konnte festgestellt werden, daß die zuvor beschriebene erfindungsgemäße Zusammensetzung hervorragend geeignet ist, Tätowierungen jeglicher Art, wie sie beispielhaft in der Einleitung zur vorliegenden Anmeldung beschrieben sind, schnell und schmerzfrei zu entfernen, ohne daß hierbei, wie bei den operativen Methoden oder der Laserbehandlung des Standes der Technik, die Gefahr besteht, daß häßliche Narben zurückbleiben. Selbst bei sehr tiefen, oftmals mehrfarbigen Tätowierungen ließen sich diese, ggf. nach Wiederholung der Anwendung der erfindungsgemäßen Zusammensetzung, restlos und insbesondere auch schmerzfrei entfernen, so daß nach einer Abheilzeit von wenigen Tagen bis zu vier Wochen ein unvoreingenommener Beobachter nicht mehr feststellen konnte, daß an dem mit der erfindungsgemäßen Zusammensetzung behandelten Hautbereich ehemals eine Tätowierung vorhanden war.

Die zuvor beschriebenen Vorteile der erfindungsgemäßen Zusammensetzung werden darauf zurückgeführt, daß offensichtlich die in der erfindungsgemäßen Zusammensetzung zwangsläufig enthaltenen Inhaltsstoffe eine synergistische Wirkung dahingehend besitzen, daß einerseits die erfindungsgemäße Zusammensetzung keinerlei Allergien hervorruft, wodurch die Schmerzlosigkeit bei der Anwendung der erfindungsgemäßen Zusammensetzung erklärlich wird, und andererseits keine Entzündungen des mit der erfindungsgemäßen Zusammensetzung behandelten Hautbereiches auftritt, wobei diese entzündungsfreie Anwendung insbesondere auch damit begründet wird, daß die erfindungsgemäße Zusammensetzung Wasserstoffperoxid in entsprechenden Konzentrationen enthält. Insbesondere konnte auch bei Anwendung der erfindungsgemäßen Zusammensetzung beobachtet werden, daß bereits nach einer kurzen Einwirkzeit die Farbpigmente der Tätowierung von der Haut abgestoßen und quasi von innen her durch das hauteigene Flüssigkeitssystem ausgeschwemmt werden, wobei sich dieser Ausschwemmvorgang so lange fortsetzt, bis sich eine das Ausschwemmen beeinflussende Schorfbildung einstellt, was jedoch erst nach einigen Stunden oder wenigen Tagen eintritt. Von daher bietet es sich auch an, den Hautbereich nach Applikation der erfindungsgemäßen Zusammensetzung und nach Beginn der Ausschwemmung mit einem System, so zum Beispiel aus Mullbinden, abzudecken, das einerseits eine unerwünschte frühzeitige Schorfbildung verhindert und andererseits zusätzlich noch aufgrund einer gewissen Kapillarität den Ausschwemmvorgang unterstützt.

Um das Eindringen der erfindungsgemäßen Zusammensetzung in den zu behandelnden Hautbereich, der mit der Tätowierung versehen ist, zu beschleunigen, ist es wünschenswert, daß dieser Hautbereich zuvor enthaart, desinfiziert und desweiteren so weit behandelt wird, daß zumindestens ein Teil der Epidermis und vorzugsweise der Hornhaut entfernt wird, wobei nachfolgend noch bei dem erfindungsgemäßen Verfahren dargelegt ist, welche bevorzugten Behandlungsmethoden hierfür verwendet werden.

Durch Variation der Konzentration der Inhaltsstoffe, die zwingender Bestandteil der erfindungsgemäßen Zusammensetzung sind und die vorstehend unter den Punkten a) bis g) aufgeführt wurden, läßt sich einerseits die Geschwindigkeit der Entfernung der jeweiligen Tätowierung und andererseits abhängig von der Tiefe und Farbigkeit der Tätowierung, die Anzahl der notwendigen Applikationen der erfindungsgemäßen Zusammensetzung variieren. Bei leichten Tätowierungen, d.h. die sich durch eine geringe Fläche der Tätowierung selbst und/oder durch die Farbtiefe der Tätowierung auszeichnen, wählt man grundsätzlich solche Konzentrationsbereiche aus, wie sie nachstehend für die Inhaltsstoffe b) bis g) durch den unteren Konzentrationswert angegeben sind, so daß hierbei die Wasserkonzentration relativ hoch ist, während für sehr farbintensive Tätowierungen und/oder für sehr großflächige Tätowierungen solche Konzentrationen der Inhaltsstoffe b) bis g) vorgezogen werden, wie sie nachfolgend durch die jeweils höheren Konzentrationen der Inhaltsstoffe b) bis g) beschrieben sind.

Unter den zuvor wiedergegebenen Gesichtspunkten weist somit im allgemeinen die erfindungsgemäße Zusammensetzung die Inhaltsstoffe a) bis g) in einem bevorzugten Konzentrationsbereich zwischen

| | | | |
|---|---|---|---|
| a) | 2 und | 9,5 % | Wasser, |
| b) | 24 und | 40 % | Milchsäureethylester, |
| c) | 0,18 und | 0,55 % | 4-Hydroxy-2-nonenal (HNE) und/oder |
| | 2 und | 12 % | d-Erythro-2-aminooctadec-4-en-1,3-diol und/oder |
| | 1 und | 3 % | Trihydroxypalmitamidohydroxypropyl Myristyl Ester, |
| d) | 29 und | 39 % | 4-Hydroxybutyl-Propionsäure, |
| e) | 12 und | 25 % | 2-Hydroxybutyl-Propionsäure, |
| f) | 0,8 und | 5,5 % | 2-Trihydroxypurin sowie |
| g) | 2,5 und | 17 % | Wasserstoffperoxid |

auf.

Weist die erfindungsgemäße Zusammensetzung eine Mischung auf, die als alleiniger Inhaltsstoff c) die Verbindung 4-Hydroxy-2-nonenal (HNE) enthält, so hat sich als besonders vorteilhaft und vielseitig anwendbar eine Zusammensetzung ergeben, die bezüglich der qualitativen und quantitativen Angaben folgende Inhaltsstoffe enthält:

| | | | |
|---|---|---|---|
| a) | 4 bis | 7,5 % | Wasser, |
| b) | 28 bis | 36 % | Milchsäureethylester, |
| c) | 0,2 bis | 0,5 % | 4-Hydroxy-2-nonenal (HNE), |
| d) | 31,5 bis | 37 % | 4-Hydroxybutyl-Propionsäure, |
| e) | 14 bis | 22,5 % | 2-Hydroxybutyl-Propionsäure, |
| f) | 1 bis | 5 % | 2-Trihydroxypurin sowie |
| g) | 7 bis | 15 % | Wasserstoffperoxid |

Ist hingegen als alleiniger Inhaltsstoff c) das d-Erythro-2-aminooctadec-4-en-1,3-diol vorhanden, so weist eine für diese Zusammensetzung besonders vorteilhafte Ausführungsform die Inhaltsstoffe a) bis g) in folgenden Konzentrationsbereichen auf, wobei es bei dieser weiteren Ausbildung der erfindungsgemäßen Zusammensetzung erfahrungsgemäß genügt, hier im Vergleich zu der zuvor beschriebenen Ausgestaltung der erfindungsgemäßen Zusammensetzung die Wasserstoffperoxid-Konzentration entsprechend zu reduzieren. Somit umfaßt diese Ausführungsform der erfindungsgemäßen Zusammensetzung folgende Inhaltsstoffe

| | | | |
|---|---|---|---|
| a) | 4 bis | 7,5 % | Wasser, |
| b) | 28 bis | 36 % | Milchsäureethylester, |
| c) | 3 bis | 10 % | d-Erythro-2-aminooctadec-4-en-1,3-diol, |
| d) | 31,5 bis | 37 % | 4-Hydroxybutyl-Propionsäure, |
| e) | 14 bis | 22,5 % | 2-Hydroxybutyl-Propionsäure, |
| f) | 1 bis | 3 % | 2-Trihydroxypurin sowie |
| g) | 3,5 bis | 10 % | Wasserstoffperoxid |

Eine weitere Möglichkeit zur Variation der Konzentration der zuvor genannten Inhaltsstoffe b) bis g) der erfindungsgemäßen Zusammensetzung wird dadurch gegeben, daß hierbei nicht etwa die konzentrierten Inhaltsstoffe selbst sondern entsprechend verdünnte Lösungen derselben zusammengemischt werden, wobei der Inhaltsstoff b), d.h. der Milchsäureethylester, insbesondere als eine 1 bis 3 Gew.%ige und vorzugsweise als eine 1,8 Gew.%ige Lösung, der Inhaltsstoff c), d.h. die 4-Hydroxybutyl-Propionsäure, als eine 14 bis 22 Gew.%ige Lösung und vorzugsweise als eine 18 Gew.%ige Lösung, der Inhaltsstoff e), d.h. die 2-Hydroxybutyl-Propionsäure, als eine 7 bis 13 Gew.%ige und vorzugsweise als eine 10 Gew.%ige Lösung und der Inhaltsstoff g), d.h. das Wasserstoffperoxid, als eine 16 bis 24 Gew.%ige und vorzugsweise als eine 20 Gew.%ige Lösung verwendet werden.

Die zweite, ebenfalls besonders im Hinblick auf die Entfernung von Tätowierungen geeignete Variante der erfindungsgemäßen Zusammensetzung unterscheidet sich von dem zuvor beschriebenen Ausführungsformen der ersten Variante der erfindungsgemäßen Zusammensetzung grundsätzlich dadurch, daß hier unterschiedliche Inhaltsstoffe vorhanden sind. Alle übrigen, zuvor beschriebenen Eigenschaften gelten auch für die nachfolgend beschriebene zweite Variante der erfindungsgemäßen Zusammensetzung, wie diese im Patentanspruch 9 definiert ist. Hierbei enthält diese Variante der erfindungsgemäßen Zusammensetzung die Inhaltsstoffe
a) Wasser,
b) Ethyl-(S)-2-hydroxypropionat,
c) 2-Hydroxypropionsäure,
d) (S)-(-)-Milchsäurebutylester und
e) Wasserstoffperoxid,
wobei auch diese Variante der erfindungsgemäßen Zusammensetzung eine flüssige bis gelartige Konsistenz besitzt, insbesondere in dem Viskositätsbereich, wie dieser vorstehend für die erste Variante der erfindungsgemäßen Zusammensetzung spezifiziert wurde. Auch liegt der pH-Wert der zweiten Variante der erfindungsgemäßen Zusammensetzung insbesondere in dem Bereich, wie dieser zuvor für die erste Variante, die durch den Patentanspruch 1 konkretisiert ist, beschrieben ist.

Die Vorteile dieser zweiten Variante unterscheiden sich nicht von den Vorteilen der ersten Variante der erfindungsgemäßen Zusammensetzung. Jedoch besitzt die zweite Variante der erfindungsgemäßen Zusammensetzung im Vergleich zur zuvor beschriebenen ersten Variante der erfindungsgemäßen Zusammensetzung den weiteren Vorteil, daß hierbei die Anzahl der Inhaltsstoffe geringer ist, so daß hierdurch eine mögliche Gefahr des Auftretens von allergischen Hautreaktionen weiter verringert ist.

Durch Variation der Konzentration der Inhaltsstoffe, die zwingender Bestandteil der zweiten Variante der erfindungsgemäßen Zusammensetzung sind und die vorstehend unter den Punkten a) bis e) aufgeführt wurden, läßt sich einerseits die Geschwindigkeit der Entfernung der jeweiligen Tätowierung und andererseits abhängig von der Tiefe und Farbigkeit der Tätowierung, die Anzahl der notwendigen Applikationen der zweiten Variante der erfindungsgemäßen Zusammensetzung variieren. Bei leichten Tätowierungen, d.h. die sich durch eine geringe Fläche der Tätowierung selbst und/oder durch die Farbtiefe der Tätowierung auszeichnen, wählt man grundsätzlich solche Konzentrationsbereiche aus, wie sie nachstehend für die Inhaltsstoffe b) bis e) durch den unteren Konzentrationswert angegeben sind, so daß hierbei die Wasserkonzentration relativ hoch ist, während für sehr farbintensive Tätowierungen und/oder für sehr großflächige Tätowierungen solche Konzentrationen der Inhaltsstoffe b) bis e) vorgezogen werden, wie sie nachfolgend durch die jeweils höheren Konzentrationen der Inhaltsstoffe b) bis e) beschrieben sind.

Unter den zuvor wiedergegebenen Gesichtspunkten weist somit im allgemeinen die erfindungsgemäße Zusammensetzung in der zweiten Variante die Inhaltsstoffe a) bis e) in einem bevorzugten Konzentrationsbereich zwischen

| | | | |
|---|---|---|---|
| a) | 14 und | 20 % | Wasser, |
| b) | | 9 und 15 % | Ethyl-(S)-2-hydroxypropionat, |
| c) | | 35 und 44 % | 2-Hydroxypropionsäure, |
| d) | | 5 und 14 % | (S)-(-)-Milchsäurebutylester und |
| e) | | 18 und 28 % | Wasserstoffperoxid |

und insbesondere die Inhaltsstoffe a) bis e) in einem besonders bevorzugten Konzentrationsbereich zwischen

| | | |
|---|---|---|
| a) | 16 und 18 % | Wasser, |
| b) | 11 und 13 % | Ethyl-(S)-2-hydroxypropionat, |
| c) | 38 und 40 % | 2-Hydroxypropionsäure, |
| d) | 8 und 10 % | (S)-(-)-Milchsäurebutylester und |
| e) | 22 und 24 % | Wasserstoffperoxid |

auf.

Bezüglich des der in der zweiten Variante der erfindungsgemäßen Zusammensetzung enthaltenen Ethyl-(S)-2-hydroxypropionat ist festzuhalten, daß dieses Ethyl-(S)-2-hydroxypropionat vorzugsweise eine Konzentration zwischen 95 und 99 % aufweist, während vorzugsweise die zweite Variante der erfindungsgemäßen Zusammensetzung das Wasserstoffperoxid als eine 16 bis 24 Gew.%ige wäßrige Lösung enthält.

Eine besonders geeignete Weiterbildung der zuvor beschriebenen Ausführungsformen der erfindungsgemäßen Zusammensetzung, die insbesondere eine hohe Lagerstabilität, verbunden mit einem geringen Abbau der Wasserstoffperoxid-Konzentration, aufweist, sieht vor, daß hierbei die zuvor beschriebenen Inhaltsstoffe von dem Wasserstoffperoxid getrennt verpackt sind. Dadurch wird wirksam erreicht, daß selbst bei einem recht sauren pH-Wert ein Zerfall des Wasserstoffperoxids, insbesondere auch bei hohen Lagertemperaturen, nicht auftreten kann, da das Wasserstoffperoxid mit den übrigen Inhaltsstoffen der Zusammensetzung erst unmittelbar vor der Anwendung vermischt wird.

Eine andere Möglichkeit zur Stabilisierung des Wasserstoffperoxids ist bei den Ausführungsformen der erfindungsgemäßen Zusammensetzung vorgesehen, bei denen die erfindungsgemäße Zusammensetzung neben den zuvor beschriebenen Inhaltsstoffen desweiteren mindestens einen Stabilisator für das Wasserstoffperoxid und/oder mindestens ein Komplexierungsmittel aufweist, wobei das Komplexierungsmittel, bei dem es sich insbesondere um Ethylendiamintetraessigsäure und vorzugsweise um das Natriumsalz von Ethylendiamintetraessigsäure handelt, sicherstellt, daß insbesondere solche Metallionen bzw. Metalle, die den Wasserstoffperoxid-Zerfall katalysieren, hierdurch komplexiert werden. Dies wiederum führt zu einer weiteren Stabilisierung der erfindungsgemäßen Zusammensetzung insbesondere bei ihrer Lagerung.

Besonders geeignete Stabilisatoren, die in der erfindungsgemäßen Zusammensetzung bei den zuvor beschriebenen Ausführungsformen vorgesehen sind, werden aus der Gruppe ausgewählt, die Phenacetin, Phosphorsäure, insbesondere Ortho-Phosphorsäure, α-Bisabolol, Terpene, Thymol, Menthol und Campher sowie Derivate der zuvor genannten Stoffe umfaßt.

Bezüglich der Konzentration der zuvor genannten Stabilisatoren für das Wasserstoffperoxid ist allgemein festzuhalten, daß sich deren Konzentration nach der erwünschten Stabilisierung und dem jeweiligen chemischen Aufbau des Stabilisators richtet. Weist insbesondere die erfindungsgemäße Zusammensetzung einen Stabilisator auf der Basis von Phenacetin oder einem Phenacetin-Derivat, so insbesondere Paracetamol, auf, so beträgt die Konzentration dieses speziellen Stabilisators in der erfindungsgemäßen Zusammensetzung vorzugsweise bis 0,5 % variiert und insbesondere zwischen 0,001 % und 0,2 %, während bei Auswahl von Phosphorsäure, insbesondere Ortho-Phosphorsäure und/oder Phosphorsäure-Derivaten, die Stabilisatorkonzentration in der erfindungsgemäßen Zusammensetzung insbesondere bis 3 % und vorzugsweise zwischen 0,3 % und 2 % beträgt.

Eine weitere Stabilisierung insbesondere in bezug auf eine lange Lagerung lassen sich bei der erfindungsgemäßen Zusammensetzung dadurch erreichen, daß das Wasserstoffperoxid zusammen mit den zuvor beschriebenen Stabilisatoren und/oder Komplexierungsmittel vermischt und getrennt von den übrigen Inhaltsstoffen der erfindungsgemäßen Zusammensetzung verpackt wird. Dementsprechend werden bei dieser hoch lagerstabilen Zusammensetzung erst unmittelbar vor ihrer Anwendung die beiden getrennt verpackten Inhaltsstoffe der Zusammensetzung miteinander vermischt.

Klarstellend sei angemerkt, daß alle in der vorliegenden Beschreibung angegebenen Konzentrationsangaben als Gewichtsprozent konkretisiert sind, es sei denn, daß ausdrücklich darauf hingewiesen wird, daß es sich hierbei um Volumenprozent handelt. Desweiteren beziehen sich alle prozentuale Konzentrationsangaben auf die anwendungsfertige Zusammensetzung, wie diese zur Entfernung der Tätowierung auf den diesbezüglichen Hautbereich aufgetragen wird.

Wasser im Sinne der vorliegenden Anmeldung umfaßt alle wäßrigen Systeme, die physiologisch unbedenklich und zugelassen sind und deckt neben destilliertem Wasser, entionisiertem Wasser, hochreinem Wasser auch solche wäßrigen Systeme ab, die zur Korrektur des pH-Wertes entsprechende Puffersysteme enthalten oder die Salze, insbesondere Kochsalz, aufweisen. Desweiteren ist festzuhalten, daß Salze oder Derivate der vorstehend unter den Merkmalen b) bis g) aufgeführten Inhaltsstoffe ebenfalls unter die jeweiligen Inhaltsstoffe selbst fallen, wobei der Begriff 2-Trihydroxypurin auch insbesondere das 2,6,8-Trihydroxypurin und der Begriff Trihydroxypalmitamidohydroxypropyl Myristyl Ester ebenfalls die Verbindung Trihydroxypalmitamidohydroxypropyl Myristyl Ether, abdecken sollen.

Der im vorliegenden Text verwendete Begriff "und/oder" bedeutet, daß die einzelnen Elemente bei der entsprechenden Aufzählung sowohl additiv oder alternativ zu sehen sind, wobei bei der additiven Benennung dann mindestens zwei Elemente der Aufzählung miteinander kombiniert sind.

Vorzugsweise weist die erfindungsgemäße Zusammensetzung einen pH-Wert auf, der im Bereich von pH 7 liegt, wobei dieser Bereich insbesondere einen pH-Wert zwischen 5,3 bis 7,9 abdeckt.

Um bei der erfindungsgemäßen Zusammensetzung eine gelartige Konsistenz sicherzustellen, bietet es sich an, hier die üblichen, im kosmetischen Bereich verwendeten Gelbildner vorzusehen, so daß die Viskosität, gemessen bei 20 °C, dieser Ausführungsformen der erfindungsgemäßen Zusammensetzung vorzugsweise zwischen 5 mPas und 40.000 mPas und insbesondere zwischen 100 mPas und 3.000 mPas variiert. Falls erforderlich, kann die erfindungsgemäße Zusammensetzung desweiteren mindestens ein Konservierungsmittel sowie die im kosmetischen Bereich üblichen Zusatzstoffe enthalten.

Die vorliegende Erfindung betrifft desweiteren ein Verfahren zur Entfernung einer Tätowierung, wobei das erfindungsgemäße Verfahren vorsieht, daß der mit der Tätowierung versehene Hautbereich nach Enthaarung und/oder Desinfektion derart behandelt wird, daß mindestens ein Teil der Epidermis und vorzugsweise der obere Teil der Epidermis und damit auch der obere Teil der Hornhaut (Stratum corneum) entfernt und hiernach auf den so vorbehandelten Hautbereich eine Zusammensetzung aufgetragen wird, wie die vorstehend als erfindungsgemäße Zusammensetzung beschrieben ist, wobei die erfindungsgemäße Zusammensetzung für eine vorgegebene Zeit einwirken gelassen wird.

Das erfindungsgemäße Verfahren hat identisch oder analog alle die Vorteile, wie sie vorstehend für die beiden Varianten der erfindungsgemäßen Zusammensetzungen ausführlich beschrieben sind. Insbesondere ist jedoch zum erfindungsgemäßen Verfahren festzuhalten, daß das erfindungsgemäße Verfahren besonders einfach anzuwenden ist, wobei die Anwendung des erfindungsgemäßen Verfahrens nicht zwangsweise die Tätigkeit eines Mediziners oder Dermatologens erfordert, sondern auch durch entsprechend geschulte Kräfte, so beispielsweise Tätowierer oder Kosmetikerinnen, erfolgen kann, da es sich bei dem erfindungsgemäßen Verfahren nicht um ein chirurgisches oder therapeutischen und somit auch nicht um ein medizinisches Verfahren handelt.

Um, wie bereits zuvor, bei der erfindungsgemäßen Zusammensetzung kurz angesprochen, das Eindringen der erfindungsgemäßen Zusammensetzung in den jeweils zu behandelnden Hautbereich, der mit der zu entfernenden Tätowierung versehen ist, zu erleichtern, sieht eine erste Weiterbildung des erfindungsgemäßen Verfahrens vor, daß hierbei der Hautbereich punktuell und/oder kleinflächig mechanisch behandelt wird. Eine punktuelle Behandlung dieses Hautbereiches, durch den der obere Bereich der Epidermis zumindest teilweise perforiert wird, liegt dann vor, wenn für diese mechanische Behandlung ein Hilfsmittel, beispielsweise eine Nadel oder ein geeigneter rotierender Fräskopf, verwendet wird, wobei die Größe einer hiermit erzeugten Fläche kleiner als 2 mm² ist, so daß entsprechend die Epidermis in ihrem äußeren Bereich mit sehr kleinen Löchern punktuell perforiert wird. Wird eine kleinflächige Behandlung des Hautbereiches ausgeführt, so bietet es sich hierfür an, die Größe der jeweils hiermit erzeugten Fläche zwischen 2 mm² und 15 mm² zu variieren, wobei insbesondere dann der Abstand zwischen benachbarten Flächen so gewählt wird, daß hierbei dieser Abstand zwischen 2 mm und 15 mm beträgt.

Erstaunlicherweise genügt es bei der Anwendung des erfindungsgemäßen Verfahrens, wenn dabei die entsprechende mechanische Behandlung bis zu einer Tiefe zwischen 0,05 mm und 0,2 mm durchgeführt wird, wobei die Tiefe selbstverständlich davon abhängt, an welchem Körperbereich die jeweils zu entfernende Tätowierung positioniert worden ist. Bei einer derartig geringen Tiefe wird dementsprechend bei dem erfindungsgemäßen Verfahren nur die Hornhaut (Stratum corneum) entsprechend teilweise perforiert, wobei über diese Perforation die erfindungsgemäße Zusammensetzung zur eigentlichen Tätowierung sehr schnell penetriert und dort den zuvor angesprochenen Ausschwemmvorgang bewirkt.

Insbesondere wird bei dem erfindungsgemäßen Verfahren als mechanische Behandlung ein Fräsen des Hautbereiches durchgeführt, wobei hier vorzugsweise mit Hochgeschwindigkeitsfräsern gearbeitet wird, die mit einem Diamant- oder Blaudiamant-Fräskopf versehen sind, wobei insbesondere der Fräskopf mit einer Drehzahl zwischen 220.000 U/min bis 270.000 U/min rotiert.

Eine weitere Möglichkeit zur Behandlung des Hautbereiches bei dem erfindungsgemäßen Verfahren wird dadurch zur Verfügung gestellt, daß hierbei der mit der Tätowierung versehene Hautbereich vollflächig geschliffen wird, wobei hierfür geeignete Schleifmittel, so zum Beispiel Schleifgewebe oder auch Schleifpasten, die üblicherweise als Peeling-Pasten bezeichnet werden, zur Anwendung gelangen. Die Tiefe dieses Beschleifens variiert vorzugsweise zwischen 0,05 mm und 0,2 mm, so daß selbst bei dem vollflächigen Beschleifen eine Narbenbildung ausgeschlossen ist.

Eine weitere Möglichkeit, um bei dem erfindungsgemäßen Verfahren das Eindringen der erfindungsgemäßen Zusammensetzungen zu erleichtern und zu beschleunigen, ist dadurch gegeben, daß der mit der Tätowierung versehene Hautbereich oberflächlich mit Mikroschnitten, d.h. somit linienförmig, versehen wird, wobei die Tiefe derartiger Mikroschnitte zwischen 0,05 mm und 0,2 mm vorzugsweise variiert. Ebenso ist es bei dem erfindungsgemäßen Verfahren möglich, den Hautbereich, der die zu entfernende Tätowierung aufweist, punktuell, linienförmig, kleinflächig oder aber auch insbesondere vollflächig, chemisch zu behandeln, wobei durch eine derartige chemische Behandlung insbesondere der obere Bereich der Hornhaut aufgequollen bzw. entfernt wird. Hierfür können beispielsweise verdünnte alkalische Lösungen, insbesondere alkalische wäßrige Lösungen und/oder tensidische Lösungen verwendet werden, wobei dieser Vorgang noch dadurch zu beschleunigen ist, daß hier eine okklusive Verbandstechnik zur Entfernung dieses Hautbereiches angewandt wird.

Eine andere Möglichkeit zur Behandlung des mit der Tätowierung versehenen Hautbereiches ist dadurch gegeben, daß bei dem erfindungsgemäßen Verfahren eine thermische Behandlung, insbesondere eine thermische Behandlung mit Hilfe von durch Hochfrequenzstrom erhitzte Nadeln, vorgesehen wird, um hierdurch den äußeren Hautbereich bis zu einer Tiefe von vorzugsweise zwischen 0,05 mm und 0,2 mm zu entfernen und somit das Penetrieren der erfindungsgemäßen Zusammensetzungen in den Tätowierungsbereich und das Ausschwemmen der Farbstoffe bzw. pigmente zu fördern.

Bezüglich der beim erfindungsgemäßen Verfahren festgelegten vorgegebenen Zeit, die nach Eindringen der erfindungsgemäßen Zusammensetzungen in den Tätowierungsbereich und dem sich hieran anschließenden Ausschwemmen anschließt, ist allgemein festzuhalten, daß sich diese Zeit einerseits nach der Tiefe der Tätowierung und andererseits der Farbigkeit der Tätowierung ausrichtet und zudem noch davon abhängt, wie großflächig die Tätowierung ist. Überraschend konnte festgestellt werden, daß insbesondere eine Zeit zwischen 5 Minuten und 60 Minuten ausgewählt wird, um schon einen guten Effekt bezüglich der Entfernung der Tätowierung zu erreichen, wobei nach Ablauf dieser Zeit, in der die erfindungsgemäßen Zusammensetzungen gegebenenfalls nochmals appliziert werden, der Überschuß der auf dem Hautbereich verbleibenden erfindungsgemäßen Zusammensetzungen abgewaschen wird und hiernach den durch das erfindungsgemäße Verfahren behandelte Hautbereich verbunden wird, wobei, falls erforderlich, das erfindungsgemäße Verfahren jeweils in einem bevorzugten Abstand von drei bis sechs Wochen, mehrfach wiederholt werden kann.

Vorteilhafte Weiterbildungen der erfindungsgemäßen Zusammensetzungen und des erfindungsgemäßen Verfahrens sind in den Unteransprüchen angegeben. Die erfindungsgemäßen Zusammensetzungen werden nachfolgend anhand von drei Ausführungsbeispielen näher erläutert.

### Ausführungsbeispiel 1

Es wurde eine Zusammensetzung I erstellt, die die nachfolgenden Inhaltsstoffe aufwies, wobei die Konzentrationen alle in Gewichtsprozent ausgewiesen sind:

| | | |
|---|---|---|
| a) | 6,5 % | Wasser, ultrarein DAB7 |
| b) | 31 % | Milchsäureethylester, |
| c) | 0,5 % | 4-Hydroxy-2-nonenal (HNE), |
| d) | 28 % | 4-Hydroxybutyl-Propionsäure, |
| e) | 20 % | 2-Hydroxybutyl-Propionsäure, |
| f) | 2 % | 2-Trihydroxypurin sowie |
| g) | 12 % | Wasserstoffperoxid |

Der Milchsäureethylester war eine 1,8 %ige Lösung, die 4-Hydroxybutyl-Propionsäure war eine 18 %ige Lösung, die 2-Hydroxy-butyl-Propionsäure war eine 10 %ige Lösung, während das Wasserstoffperoxid eine 20 %ige wäßrige Lösung war.

Zur Herstellung der Zusammensetzung I wurden die zuvor aufgeführten Komponenten in einem hochtourigem Rührer (1.000 U/min) bei Raumtemperatur so lange vermischt, bis eine homogene flüssige Mischung mit einer wäßrigen Konsistenz entstand.

Die so hergestellte Zusammensetzung I zeigte auch nach einer Standzeit von 12 Wochen keine Entmischung.

Eine Probandin, die am rechten Oberarm mit einer, einen Salamander darstellenden und etwa 10 cm langen Tätowierung versehen war, die primär die Farben blau und rot aufwies, wurde nach vorheriger Rasur der Behaarung und Desinfektion des mit der Tätowierung versehenen Hautbereiches wie folgt mit der Zusammensetzung I behandelt:
Zunächst wurde mit einem Hochgeschwindigkeitsfräser mit einem rotierenden Diamantfräskopf die die Tätowierung abdeckende Haut punktuell abgefräst, wobei hierfür der Fräser eine Drehzahl von 250.000 Umdrehungen/min aufwies. Der Abstand der etwa 1 mm² großen Fräslöcher im Hautbereich, der oberhalb der Tätowierung liegt, betrug allseitig 10 mm, so daß ein symmetrisches Lochmuster entstand. Die Frästiefe wurde auf 0,15 mm eingestellt.

Auf den so behandelten Hautbereich wurde die Zusammensetzung I aufgetragen, wobei darauf geachtet wurde, daß der behandelte Hautbereich stets mit der Zusammensetzung I feucht gehalten wurde. Nach einer Verweilzeit, d.h. einer vorgegebenen Zeit von 50 Minuten, und nachdem dabei bereits Farbpigmente ausgespült waren, wurde der Hautbereich mit sterilem Wasser abgewaschen und hiernach mit einer Mullbinde abgedeckt, die wiederholt während der nächsten drei Tage gewechselt wurde. Hierbei konnte festgestellt werden, daß durch die Fräslöcher permanent Farbpigmente ausgeschwemmt wurden.

Bereits nach Ablauf von drei Tagen war die so behandelte Tätowierung deutlich in der Fläche und der Kontur verringert, d.h. es zeigten sich schon Bereiche, in denen keine Farbpigmente mehr vorhanden waren.

Die zuvor beschriebene Behandlung wurde nach drei Wochen und nach sechs Wochen, jeweils berechnet ab erster Behandlung, identisch wiederholt.

Nach acht Wochen, gerechnet ab Beginn der Behandlung, wurden fünf unvoreingenommene Gutachter dahingehend beauftragt, den ehemals tätowierten Hautbereich an dem Arm der Probandin zu lokalisieren. Keiner der fünf Gutachter war in der Lage, den ehemals tätowierten Bereich korrekt zu bestimmen. Narben oder sonstige Veränderungen der Haut im ehemaligen Tätowierungsbereich konnten ebenfalls nicht aufgefunden werden.

Weder bei der Behandlung noch im Anschluß hieran berichtete die Probandin über Schmerzen. Ebensowenig trat eine Entzündung des behandelten Hautbereiches auf.

### Ausführungsbeispiel 2

Es wurde eine Zusammensetzung II erstellt, die die nachfolgenden Inhaltsstoffe aufwies, wobei die Konzentrationen alle in Gewichtsprozent ausgewiesen sind:

| | | |
|---|---|---|
| a) | 5,9 % | Wasser, ultrarein DAB 7 |
| b) | 29,5 % | Milchsäureethylester, |
| c) | 3,5 % | d-Erythro-2-aminooctadec-4-en-1,3-diol, |
| d) | 34,3 % | 4-Hydroxybutyl-Propionsäure, |
| e) | 14,5 % | 2-Hydroxybutyl-Propionsäure, |
| f) | 2,3 % | 2-Trihydroxypurin sowie |
| g) | 10 % | Wasserstoffperoxid |

Der Milchsäureethylester war eine 1,8 %ige Lösung, die 4-Hydroxybutyl-Propionsäure war eine 18 %ige Lösung, die 2-Hydroxybutyl-Propionsäure war eine 10 %ige Lösung, während das Wasserstoffperoxid eine 20 %ige wäßrige Lösung war.

Zur Herstellung der Zusammensetzung II wurden die zuvor aufgeführten Komponenten in einem hochtourigem Rührer (1.000 U/min) bei Raumtemperatur so lange vermischt, bis eine homogene flüssige Mischung mit einer wäßrigen Konsistenz entstand.

Die so hergestellte Zusammensetzung II zeigte auch nach einer Standzeit von zwei Monaten keine Entmischung.

Ein Proband, der am linken Unterarm mit einer, ein Herz und einen dieses durchbrechenden Pfeil darstellenden und etwa ca. 8 cm langen und ca. 4 cm breiten Tätowierung versehen war, die primär die Farbe schwarz aufwies, wurde nach vorheriger Rasur der Behaarung und Desinfektion des mit der Tätowierung versehenen Hautbereiches wie folgt mit der Zusammensetzung II behandelt:
Mit Hilfe eines kommerziellen Epiliergerätes des Typs Danycare, T-Away wurde mittels einer Nadel, die über einen hochfrequenten Strom permanent erhitzt war, die Haut in dem die Tätowierung abdeckenden Hautbereich linienförmig (Breite ca. 2 mm) schichtweise abgetragen, wobei die Abtragtiefe etwa 0,15 mm betrug.

Auf den so behandelten Hautbereich wurde die Zusammensetzung II aufgetragen, wobei darauf geachtet wurde, daß der behandelte Hautbereich stets mit der Zusammensetzung II feucht gehalten wurde. Nach einer Verweilzeit von 30 Minuten und nachdem dabei bereits Farbpigmente ausgespült waren, wurde der Hautbereich mit sterilem Wasser abgewaschen und hiernach mit einer Mullbinde abgedeckt, die wiederholt während der nächsten zwei Tage gewechselt wurde. Hierbei konnte festgestellt werden, daß durch die linienförmigen Vertiefungen permanent Farbpigmente ausgeschwemmt wurden.

Bereits nach Ablauf von zwei Tagen war die so behandelte Tätowierung deutlich in der Fläche und der Kontur verringert, d.h. es zeigten sich schon Bereiche, in denen keine Farbpigmente mehr vorhanden waren.

Die zuvor beschriebene Behandlung wurde nach zwei Wochen und nach vier Wochen, jeweils berechnet ab erster Behandlung, identisch wiederholt.

Nach sechs Wochen, gerechnet ab Beginn der Behandlung, wurden drei unvoreingenommene Gutachter dahingehend beauftragt, den ehemals tätowierten Hautbereich an dem Arm des Probanden zu lokalisieren. Keiner der drei Gutachter war in der Lage, den ehemals tätowierten Bereich korrekt zu bestimmen. Narben oder sonstige Veränderungen der Haut im ehemaligen Tätowierungsbereich konnten ebenfalls nicht aufgefunden werden.

Weder bei der Behandlung noch im Anschluß hieran berichtete der Proband über Schmerzen. Ebensowenig trat eine Entzündung des behandelten Hautbereiches auf.

### Ausführungsbeispiel 3

Es wurde eine Zusammensetzung III erstellt, die die nachfolgenden Inhaltsstoffe aufwies, wobei die Konzentrationen alle in Gewichtsprozent ausgewiesen sind:

| | | |
|---|---|---|
| a) | 17 % | Wasser, |
| b) | 12 % | Ethyl-(S)-2-hydroxypropionat, |
| c) | 39 % | 2-Hydroxypropionsäure, |
| d) | 9 % | (S)-(-)-Milchsäurebutylester und |
| e) | 23 % | Wasserstoffperoxid |

Das Ethyl-(S)-2-hydroxypropionat lag in einer Konzentration 98 Gew.% vor, während das Wasserstoffperoxid eine 20 %ige wäßrige Lösung war.

Zur Herstellung der Zusammensetzung III wurden die zuvor aufgeführten Komponenten in einem hochtourigem Rührer (1.000 U/min) bei Raumtemperatur so lange vermischt, bis eine homogene flüssige Mischung mit einer wäßrigen Konsistenz entstand.

Die so hergestellte Zusammensetzung III zeigte auch nach einer Standzeit von zwei Monaten keine Entmischung.

Ein Proband, der am linken Oberrarm mit einer girlandenartigen ca. 12 cm langen und etwa 3 cm breiten Tätowierung versehen war, die primär die Farbe schwarz aufwies, wurde nach vorheriger Rasur der Behaarung und Desinfektion des mit der Tätowierung versehenen Hautbereiches wie folgt mit der Zusammensetzung III behandelt:
Mit Hilfe eines kommerziellen Epiliergerätes des Typs Danycare, T-Away wurde mittels einer Nadel, die über einen hochfrequenten Strom permanent erhitzt war, die Haut in dem die Tätowierung abdeckenden Hautbereich linienförmig (Breite ca. 2 mm) schichtweise abgetragen, wobei die Abtragtiefe etwa 0,15 mm betrug.

Auf den so behandelten Hautbereich wurde die Zusammensetzung III aufgetragen, wobei darauf geachtet wurde, daß der behandelte Hautbereich stets mit der Zusammensetzung III feucht gehalten wurde. Nach einer Verweilzeit von 30 Minuten und nachdem dabei bereits Farbpigmente ausgespült waren, wurde der Hautbereich mit sterilem Wasser abgewaschen und hiernach mit einer Mullbinde abgedeckt, die wiederholt während der nächsten drei Tage gewechselt wurde. Hierbei konnte festgestellt werden, daß durch die linienförmigen Vertiefungen permanent Farbpigmente ausgeschwemmt wurden.

Bereits nach Ablauf von drei Tagen war die so behandelte Tätowierung deutlich in der Fläche und der Kontur verringert, d.h. es zeigten sich schon Bereiche, in denen keine Farbpigmente mehr vorhanden waren.

Die zuvor beschriebene Behandlung wurde nach zweieinhalb Wochen und nach fünf Wochen, jeweils gerechnet ab erster Behandlung, identisch wiederholt.

Nach sieben Wochen, gerechnet ab Beginn der Behandlung, wurden drei unvoreingenommene Gutachter dahingehend beauftragt, den ehemals tätowierten Hautbereich an dem Oberarm des Probanden zu lokalisieren. Keiner der drei Gutachter war in der Lage, den ehemals tätowierten Bereich korrekt zu bestimmen. Narben oder sonstige Veränderungen der Haut im ehemaligen Tätowierungsbereich konnten ebenfalls nicht aufgefunden werden.

Weder bei der Behandlung noch im Anschluß hieran berichtete der Proband über Schmerzen. Ebensowenig trat eine Entzündung oder eine allergische Reaktion des behandelten Hautbereiches auf.

## Patentansprüche

1. Zusammensetzung zur Entfernung einer auf einem Hautbereich vorhandenen Tätowierung nach vorheriger Enthaarung, Desinfektion und einer Behandlung zum mindestens teilweise Abtragen der Epidermis im Bereich der Tätowierung, **dadurch gekennzeichnet, daß** die Zusammensetzung eine flüssige bis gelartige Konsistenz aufweist und eine Mischung aus
a) Wasser,
b) Milchsäureethylester,
c) 4-Hydroxy-2-nonenal (HNE) und/oder d-Erythro-2-aminooctadec-4-en-1,3-diol und/oder Trihydroxypalmitamidohydroxypropyl Myristyl Ester,
d) 4-Hydroxybutyl-Propionsäure,
e) 2-Hydroxybutyl-Propionsäure,
f) 2-Trihydroxypurin sowie
g) Wasserstoffperoxid
enthält.

2. Zusammensetzung nach Anspruch 1, **dadurch gekennzeichnet, daß** die Zusammensetzung zwischen
| | | | |
|---|---|---|---|
| a) | 2 und | 9,5 % | Wasser, |
| b) | 24 und | 40 % | Milchsäureethylester, |
| c) | 0,18 und | 0,55 % | 4-Hydroxy-2-nonenal (HNE) und/oder |
| | 2 und | 12 % | d-Erythro-2-aminooctadec-4-en-1,3-diol und/oder |
| | 1 und | 3 % | Trihydroxypalmitamidohydroxypropyl Myristyl Ester |
| d) | 29 und | 39 % | 4-Hydroxybutyl-Propionsäure, |
| e) | 12 und | 25 % | 2-Hydroxybutyl-Propionsäure, |
| f) | 0,8 und | 5,5 % | 2-Trihydroxypurin sowie |
| g) | 2,5 und | 17 % | Wasserstoffperoxid |
enthält.

3. Zusammensetzung nach Anspruch 1 oder 2, **dadurch gekennzeichnet, daß** die Zusammensetzung eine Mischung aus
| | | | |
|---|---|---|---|
| a) | 4 bis | 7,5 % | Wasser, |
| b) | 28 bis | 36 % | Milchsäureethylester, |
| c) | 0,2 bis | 0,5 % | 4-Hydroxy-2-nonenal (HNE), |
| d) | 31,5 bis | 37 % | 4-Hydroxybutyl-Propionsäure, |
| e) | 14 bis | 22,5 % | 2-Hydroxybutyl-Propionsäure, |
| f) | 1 bis | 5 % | 2-Trihydroxypurin sowie |
| g) | 7 bis | 15 % | Wasserstoffperoxid |
enthält.

4. Zusammensetzung nach Anspruch 1 oder 2, **dadurch gekennzeichnet, daß** die Zusammensetzung eine Mischung aus
| | | | |
|---|---|---|---|
| a) | 4 bis | 7,5 % | Wasser, |
| b) | 28 bis | 36 % | Milchsäureethylester, |
| c) | 3 bis | 10 % | d-Erythro-2-aminooctadec-4-en-1,3-diol, |
| d) | 31,5 bis | 37 % | 4-Hydroxybutyl-Propionsäure, |
| e) | 14 bis | 22,5 % | 2-Hydroxybutyl-Propionsäure, |
| f) | 1 bis | 3 % | 2-Trihydroxypurin sowie |
| g) | 3,5 bis | 10 % | Wasserstoffperoxid |
enthält.

5. Zusammensetzung nach einem der vorangehenden Ansprüche, **dadurch gekennzeichnet, daß** die Zusammensetzung den Milchsäureethylester als eine 1 bis 3 Gew.%ige Lösung enthält.

6. Zusammensetzung nach einem der vorangehenden Ansprüche, **dadurch gekennzeichnet, daß** die Zusammensetzung die 4-Hydroxybutyl-Propionsäure als eine 14 bis 22 Gew.%ige Lösung enthält.

7. Zusammensetzung nach einem der vorangehenden Ansprüche, **dadurch gekennzeichnet, daß** die Zusammensetzung die 2-Hydroxybutyl-Propionsäure als eine 7 bis 13 Gew.%ige Lösung enthält.

8. Zusammensetzung nach einem der vorangehenden Ansprüche, **dadurch gekennzeichnet, daß** die Zusammensetzung den Wasserstoffperoxid als eine 16 bis 24 Gew.%ige Lösung enthält.

9. Zusammensetzung zur Entfernung einer auf einem Hautbereich vorhandenen Tätowierung nach vorheriger Enthaarung, Desinfektion und einer Behandlung zum mindestens teilweise Abtragen der Epidermis im Bereich der Tätowierung, **dadurch gekennzeichnet, daß** die Zusammensetzung eine flüssige bis gelartige Konsistenz aufweist und eine Mischung aus
a) Wasser,
b) Ethyl-(S)-2-hydroxypropionat,
c) 2-Hydroxypropionsäure,
d) (S)-(-)-Milchsäurebutylester und
e) Wasserstoffperoxid
enthält.

10. Zusammensetzung nach Anspruch 9, **dadurch gekennzeichnet, daß** die Zusammensetzung zwischen
| | | |
|---|---|---|
| a) | 14 und 20 % | Wasser, |
| b) | 9 und 15 % | Ethyl-(S)-2-hydroxypropionat, |
| c) | 35 und 44 % | 2-Hydroxypropionsäure, |
| d) | 5 und 14 % | (S)-(-)-Milchsäurebutylester und |
| e) | 18 und 28 % | Wasserstoffperoxid |
enthält.

11. Zusammensetzung nach Anspruch 9 oder 10, **dadurch gekennzeichnet, daß** die Zusammensetzung zwischen
| | | |
|---|---|---|
| a) | 16 und 18 % | Wasser, |
| b) | 11 und 13 % | Ethyl-(S)-2-hydroxypropionat, |
| c) | 38 und 40 % | 2-Hydroxypropionsäure, |
| d) | 8 und 10 % | (S)-(-)-Milchsäurebutylester und |
| e) | 22 und 24 % | Wasserstoffperoxid |
enthält.

12. Zusammensetzung nach einem der Ansprüche 9 bis 11, **dadurch gekennzeichnet, daß** das in der Zusammensetzung enthaltene Ethyl-(S)-2-hydroxypropionat eine Konzentration zwischen 95 und 99 % aufweist.

13. Zusammensetzung nach einem der Ansprüche 9 bis 12, **dadurch gekennzeichnet, daß** die Zusammensetzung den Wasserstoffperoxid als eine 16 bis 24 Gew.%ige Lösung enthält.

14. Zusammensetzung nach einem der vorangehenden Ansprüche, **dadurch gekennzeichnet, daß** das Wasserstoffperoxid getrennt von den übrigen Inhaltsstoffen der Zusammensetzung verpackt ist.

15. Zusammensetzung nach einem der vorangehenden Ansprüche, **dadurch gekennzeichnet, daß** die Zusammensetzung mindestens einen Stabilisator für das Wasserstoffperoxid und/oder mindestens ein Komplexierungsmittel aufweist.

16. Zusammensetzung nach Anspruch 15, **dadurch gekennzeichnet, daß** der Stabilisator für das Wasserstoffperoxid aus der Gruppe ausgewählt ist, die Phenacetin, Phosphorsäure, α-Bisabolol, Terpene, Thymol, Menthol und Campher sowie Derivate der zuvor genannten Stoffe umfaßt.

17. Zusammensetzung nach Anspruch 15 oder 16, **dadurch gekennzeichnet, daß** die Zusammensetzung Phenacetin in einer Konzentration bis 0,5.%, insbesondere zwischen 0,001 % und 0,2 %, und/oder die Phosphorsäure in einer Konzentration bis 3 %, insbesondere zwischen 0,3 % und 2 %, aufweist.

18. Nicht-therapeutisches Verfahren zur Entfernung einer Tätowierung, bei dem der mit der Tätowierung versehene Hautbereich nach Enthaarung und/oder Desinfektion derart behandelt wird, daß zumindest ein Teil der Epidermis entfernt und hiernach auf den Hautbereich eine Zusammensetzung nach einem der Ansprüche 1 bis 17 aufgetragen und für eine vorgegebene Zeit einwirken gelassen wird.

19. Verfahren nach Anspruch 18, **dadurch gekennzeichnet, daß** die Behandlung des Hautbereiches punktuell und/oder kleinflächig mechanisch durchgeführt wird.

20. Verfahren nach Anspruch 18 oder 19, **dadurch gekennzeichnet, daß** die mechanische Behandlung des Hautbereiches kleinflächig ausgeführt wird, wobei die Größe einer jeden Fläche zwischen 2 mm² und 15 mm² variiert.

21. Verfahren nach einem der Ansprüche 18 bis 20, **dadurch gekennzeichnet, daß** der Abstand zwischen benachbarten Flächen zwischen 2 mm und 15 mm variiert.

22. Verfahren nach einem der Ansprüche 18 bis 21, **dadurch gekennzeichnet, daß** die mechanische Behandlung bis zu einer Tiefe zwischen 0,05 mm und 0,2 mm durchgeführt wird.

23. Verfahren nach einem der Ansprüche 18 bis 22, **dadurch gekennzeichnet, daß** die mechanische Behandlung durch Fräsen des Hautbereiches durchgeführt wird.

24. Verfahren nach einem der Ansprüche 18 bis 23, **dadurch gekennzeichnet, daß** als vorgegebene Zeit eine Zeit zwischen 5 Minuten und 60 Minuten ausgewählt wird.

25. Verfahren nach einem der Ansprüche 18 bis 24, **dadurch gekennzeichnet, daß** die Behandlung eine thermische Behandlung umfaßt.

26. Verfahren nach einem der Ansprüche 18, 22, 24 oder 25, **dadurch gekennzeichnet, daß** der Hautbereich vollflächig mechanisch und/oder chemisch behandelt wird.

27. Verfahren nach Anspruch 26, **dadurch gekennzeichnet, daß** der Hautbereich geschliffen wird.

## Claims

1. A composition for removing a tattoo present on a skin area after prior depilation, disinfection and a treatment to at least partially ablate the epidermis in the area of the tattoo, **characterized in that** said composition has a liquid to gel-like consistency and comprises a mixture of
a) water,
b) ethyl lactate,
c) 4-hydroxy-2-nonenal (HNE) and/or d-erythro-2-aminooctadec-4-ene-1,3-diol and/or trihydroxypalmitamidohydroxypropyl myristyl ester,
d) 4-hydroxybutyl-propionic acid,
e) 2-hydroxybutyl-propionic acid,
f) 2-trihydroxypurine and
g) hydrogen peroxide.

2. The composition according to claim 1, **characterized in that** said composition comprises between
| | | | |
|---|---|---|---|
| a) | 2 and | 9,5 % | of water, |
| b) | 24 and | 40 % | of ethyl lactate, |
| c) | 0,18 and | 0,55 % | of 4-hydroxy-2-nonenal (HNE) and/or |
| | 2 and | 12% | of d-erythro-2-aminooctadec-4-ene-1,3-diol and/or |
| | 1 and | 3 % | of trihydroxypalmitamidohydroxypropyl myristyl ester, |
| d) | 29 and | 39 % | of 4-hydroxybutyl-propionic acid, |
| e) | 12 and | 25 % | of 2-hydroxybutyl-propionic acid, |
| f) | 0,8 and | 5,5 % | of 2-trihydroxypurine and |
| g) | 2,5 and | 17 % | of hydrogen peroxide. |

3. The composition according to claim 1 or claim 2, **characterized in that** said composition comprises a mixture of
| | | | |
|---|---|---|---|
| a) | 4 to | 7,5 % | of water, |
| b) | 28 to | 36 % | of ethyl lactate, |
| c) | 0,2 to | 0,5 % | of 4-hydroxy-2-nonenal (HNE), |
| d) | 31,5 to | 37 % | of 4-hydroxybutyl-propionic acid, |
| e) | 14 to | 22,5 % | of 2-hydroxybutyl-propionic acid, |
| f) | 1 to | 5 % | of 2-trihydroxypurine and |
| g) | 7 to | 15 % | of hydrogen peroxide. |

4. The composition according to claim 1 or claim 2, **characterized in that** said composition comprises a mixture of
| | | | |
|---|---|---|---|
| a) | 4 to | 7,5 % | of water, |
| b) | 28 to | 36 % | of ethyl lactate, |
| c) | 3 to | 10 % | of d-erythro-2-aminooctadec-4-ene-1,3-diol, |
| d) | 31,5 to | 37 % | of 4-hydroxybutyl-propionic acid, |
| e) | 14 to | 22,5 % | of 2-hydroxybutyl-propionic acid, |
| f) | 1 to | 3 % | of 2-trihydroxypurine and |
| g) | 3,5 to | 10 % | of hydrogen peroxide. |

5. The composition according to any one of the previous claims, **characterized in that** said composition comprises ethyl lactate as a 1 to 3 % by weight solution.

6. The composition according to any one of the previous claims, **characterized in that** said composition comprises 4-hydroxybutyl-propionic acid as a 14 to 22 % by weight solution.

7. The composition according to any one of the previous claims, **characterized in that** said composition comprises 2-hydroxybutyl-propionic acid as a 7 to 13 % by weight solution.

8. The composition according to any one of the previous claims, **characterized in that** said composition comprises hydrogen peroxide as a 16 to 24 % by weight solution.

9. A composition for removing a tattoo present on a skin area after prior depilation, disinfection and a treatment to at least partially ablate the epidermis in the area of the tattoo, **characterized in that** said composition has a liquid to gel-like consistency and comprises a mixture of
a) water,
b) ethyl (S)-2-hydroxypropionate,
c) 2-hydroxypropionic acid,
d) (S)-(-)-butyl lactate and
e) hydrogen peroxide.

10. The composition according to claim 9, **characterized in that** said composition comprises between
| | | |
|---|---|---|
| a) | 14 and 20 % | of water, |
| b) | 9 and 15 % | of ethyl (S)-2-hydroxypropionate, |
| c) | 35 and 44 % | of 2-hydroxypropionic acid, |
| d) | 5 and 14 % | of (S)-(-)-butyl lactate and |
| e) | 18 and 28 % | of hydrogen peroxide. |

11. The composition according to claim 9 or claim 10, **characterized in that** said composition comprises between
| | | |
|---|---|---|
| a) | 16 and 18 % | of water, |
| b) | 11 and 13 % | of ethyl (S)-2-hydroxypropionate, |
| c) | 38 and 40 % | of 2-hydroxypropionic acid, |
| d) | 8 and 10 % | of (S)-(-)-butyl lactate and |
| e) | 22 and 24 % | of hydrogen peroxide. |

12. The composition according to any one of claims 9 to 11, **characterized in that** the ethyl (S)-2-hydroxypropionate included in the composition has a concentration between 95 and 99 %.

13. The composition according to any one of claims 9 to 12, **characterized in that** said composition comprises hydrogen peroxide as a 16 to 24 % by weight solution.

14. The composition according to any one of the previous claims, **characterized in that** hydrogen peroxide is packaged separately from the remaining ingredients of the composition.

15. The composition according to any one of the previous claims, **characterized in that** said composition comprises at least one stabilizer for the hydrogen peroxide and/or at least one complexing agent.

16. The composition according to claim 15, **characterized in that** the stabilizer for the hydrogen peroxide is selected from the group comprising phenacetin, phosphoric acid, α-bisabolol, terpenes, thymol, menthol and camphor as well as derivatives of the substances mentioned above.

17. The composition according to claim 15 or claim 16, **characterized in that** said composition comprises phenacetin in a concentration of up to 0,5 %, in particular between 0,001 % and 0,2 %, and/or phosphoric acid in a concentration of up to 3 %, in particular between 0,3 % and 2 %.

18. A non-therapeutic method for removing a tattoo wherein the skin area provided with the tattoo, after depilation and/or disinfection, is treated in such a way that at least a part of the epidermis is removed, and afterwards a composition according to any of claims 1 to 17 is applied to the skin area and allowed to take effect for a predetermined time period.

19. The method according to claim 18, **characterized in that** treatment of the skin area is done mechanically spot by spot and/or over small areas.

20. The method according to claim 18 or claim 19, **characterized in that** the mechanical treatment of the skin area is done over small areas, the size of each area varying between 2 mm² and 15 mm².

21. The method according to any one of claims 18 to 20, **characterized in that** the distance between adjacent areas varies between 2 mm and 15 mm.

22. The method according to any one of claims 18 to 21, **characterized in that** the mechanical treatment is done up to a depth between 0,05 mm and 0,2 mm.

23. The method according to any one of claims 18 to 22, **characterized in that** the mechanical treatment is done by abrasion of the skin area using a fraise.

24. The method according to any one of claims 18 to 23, **characterized in that** as predetermined time period, a time period between 5 minutes and 60 minutes is selected.

25. The method according to any one of claims 18 to 24, **characterized in that** the treatment comprises thermal treatment.

26. The method according to any of claims 18, 22, 24 or 25, **characterized in that** the whole skin area is treated mechanically and/or chemically.

27. The method according to claim 26, **characterized in that** the skin area is grinded.

## Revendications

1. Composition pour enlever un tatouage présent sur une zone de peau après épilation préalable, désinfection et un traitement pour l'abrasion au moins partielle de l'épiderme dans la zone du tatouage, **caractérisée en ce que** la composition présente une consistance liquide à celle d'un gel et contient un mélange
a) d'eau
b) d'ester éthylique de l'acide lactique,
c) de 4-hydroxy-2-nonénal (HNE) et/ou le d-érythro-2-aminooctadéc-4-èn-1,3-diol et/ou l'éther de trihydroxypalmitamidohydroxypropyle et de myristyle,
d) d'acide 4-hydroxybutylpropionique,
e) d'acide 2-hydroxybutylpropionique,
f) de 2-trihydroxypurine, ainsi que
g) de peroxyde d'hydrogène.

2. Composition selon la revendication 1, **caractérisée en ce que** la composition contient
a) de 2 à 9,5 % d'eau
b) de 24 à 40 % d'ester éthylique de l'acide lactique,
c) de 0,18 à 0,55 % de 4-hydroxy-2-nonénal (HNE) et/ou de 2 à 12 % de d-érythro-2-aminooctadéc-4-èn-1,3-diol et/ou 1 à 3 % d'éther de trihydroxypalmitamidohydroxypropyle et de myristyle,
d) de 29 à 39 % d'acide 4-hydroxybutylpropionique,
e) de 12 à 25 % d'acide 2-hydroxybutylpropionique,
f) de 0,8 à 5,5 % de 2-trihydroxypurine, ainsi que
g) de 2,5 à 17 % de peroxyde d'hydrogène.

3. Composition selon la revendication 1 ou 2, **caractérisée en ce que** la composition contient un mélange
a) de 4 à 7,5 % d'eau
b) de 28 à 36 % d'ester éthylique de l'acide lactique,
c) de 0,2 à 0,5 % de 4-hydroxy-2-nonénal (HNE) et/ou
d) de 31,5 à 37 % d'acide 4-hydroxybutylpropionique,
e) de 14 à 22,5 % d'acide 2-hydroxybutylpropionique,
f) de 1 à 5 % de 2-trihydroxypurine, ainsi que
g) de 7 à 15 % de peroxyde d'hydrogène.

4. Composition selon la revendication 1 ou 2, **caractérisée en ce que** la composition contient un mélange
a) de 4 à 7,5 % d'eau
b) de 28 à 36 % d'ester éthylique de l'acide lactique,
c) de 3 à 10 % de d-érythro-2-aminooctadéc-4-èn-1,3-diol,
d) de 31,5 à 37 % d'acide 4-hydroxybutylpropionique,
e) de 14 à 22,5 % d'acide 2-hydroxybutylpropionique,
f) de 1 à 3 % de 2-trihydroxypurine, ainsi que
g) de 3,5 à 10 % de peroxyde d'hydrogène.

5. Composition selon l'une quelconque des revendications précédentes, **caractérisée en ce que** la composition contient l'ester méthylique de l'acide lactique sous forme d'une solution de 1 à 3 % en poids.

6. Composition selon l'une quelconque des revendications précédentes, **caractérisée en ce que** la composition contient l'acide 4-hydroxybutylpropionique sous forme d'une solution de 14 à 22 % en poids.

7. Composition selon l'une quelconque des revendications précédentes, **caractérisée en ce que** la composition contient l'acide 2-hydroxybutylpropionique sous forme d'une solution de 7 à 13 % en poids.

8. Composition selon l'une quelconque des revendications précédentes, **caractérisée en ce que** la composition contient le peroxyde d'hydrogène sous forme d'une solution de 16 à 24 % en poids.

9. Composition pour enlever un tatouage présent sur une zone de peau après épilation préalable, désinfection et un traitement pour l'abrasion au moins partielle de l'épiderme dans la zone du tatouage, **caractérisée en ce que** la composition présente une consistance liquide à celle d'un gel et contient un mélange
a) d'eau
b) de (S)-2-hydroxypropionate d'éthyle,
c) d'acide 2-hydroxypropionique,
d) de (S)-(-)-ester butylique de l'acide lactique, et
e) de peroxyde d'hydrogène.

10. Composition selon la revendication 9, **caractérisée en ce que** la composition contient
a) de 14 à 20 % d'eau
b) de 9 à 15 % de (S)-2-hydroxypropionate d'éthyle,
c) de 35 à 44 % d'acide 2-hydroxypropionique,
d) de 5 à 14 % de (S)-(-)-ester butylique de l'acide lactique, et
e) de 18 à 28 % de peroxyde d'hydrogène.

11. Composition selon la revendication 9 ou 10, **caractérisée en ce que** la composition contient
a) de 16 à 18 % d'eau
b) de 11 à 13 % de (S)-2-hydroxypropionate d'éthyle,
c) de 38 à 40 % d'acide 2-hydroxypropionique,
d) de 8 à 10 % de (S)-(-)-ester butylique de l'acide lactique, et
e) de 22 à 24 % de peroxyde d'hydrogène.

12. Composition selon l'une quelconque des revendications 9 à 11, **caractérisée en ce que** le (S)-2-hydroxypropionate d'éthyle contenu dans la composition présente une concentration située dans l'intervalle allant de 95 à 99 %.

13. Composition selon l'une quelconque des revendications 9 à 12, **caractérisée en ce que** la composition contient le peroxyde d'hydrogène sous forme d'une solution de 16 à 24 % en poids.

14. Composition selon l'une quelconque des revendications précédentes, **caractérisée en ce que** le peroxyde d'hydrogène est présent dans un emballage séparé des autres constituants de la composition.

15. Composition selon l'une quelconque des revendications précédentes, **caractérisée en ce que** la composition présente au moins un stabilisant pour le peroxyde d'hydrogène et/ou au moins un agent de complexation.

16. Composition selon la revendication 15, **caractérisée en ce que** le stabilisant pour le peroxyde d'hydrogène est choisi dans le groupe qui comprend la phénacétine, l'acide phosphorique, l'α-bisabolol, un terpène, le thymol, le menthol et le camphre, ainsi que des dérivés des substances citées.

17. Composition selon la revendication 15 ou 16, **caractérisée en ce que** la composition contient la phénacétine en une concentration de jusqu'à 0,5 %, en particulier dans l'intervalle allant de 0,001 % à 0,2 %, et/ou l'acide phosphorique en une concentration de jusqu'à 3 %, en particulier dans l'intervalle allant de 0,3 % à 2 %.

18. Procédé non thérapeutique pour enlever un tatouage, dans lequel la zone de peau présentant un tatouage, après avoir été épilée et/ou désinfectée, est traitée que telle sorte qu'au moins une partie de l'épiderme est éliminée, puis on applique sur cette zone de peau, une composition selon l'une quelconque des revendications 1 à 17 et on laisse agir pendant une période donnée.

19. Procédé selon la revendication 18, **caractérisé en ce que** le traitement de la zone de peau est effectué de manière mécanique, ponctuellement et/ou par petites surfaces.

20. Procédé selon la revendication 18 ou 19, **caractérisé en ce que** le traitement mécanique de la zone de peau est réalisé par petites surfaces, où la taille de chaque surface varie dans l'intervalle allant de 2 mm² à 15 mm².

21. Procédé selon l'une quelconque des revendications 18 à 20, **caractérisé en ce que** la distance entre les surfaces voisines varie dans l'intervalle allant de 2 mm à 15 mm.

22. Procédé selon l'une quelconque des revendications 18 à 21, **caractérisé en ce que** le traitement mécanique est effectué jusqu'à une profondeur située dans l'intervalle allant de 0,05 mm à 0,2 mm.

23. Procédé selon l'une quelconque des revendications 18 à 22, **caractérisé en ce que** le traitement mécanique est effectué par fraisage de la zone de peau.

24. Procédé selon l'une quelconque des revendications 18 à 23, **caractérisé en ce que** comme période donnée, on choisit une période située dans l'intervalle allant de 5 minutes à 60 minutes.

25. Procédé selon l'une quelconque des revendications 18 à 24, **caractérisé en ce que** le traitement comprend un traitement thermique.

26. Procédé selon l'une quelconque des revendications 18, 22, 24 ou 25, **caractérisé en ce que** la zone de peau est traitée sur toute la surface mécaniquement et/ou chimiquement.

27. Procédé selon la revendication 26, **caractérisé en ce que** la zone de peau est abrasée.
